# EUROPÄISCHE PATENTSCHRIFT

(11) **EP 3 457 451 B1**
(45) Veröffentlichungstag und Bekanntmachung des Hinweises auf die Patenterteilung: **17.07.2019**
(21) Anmeldenummer: 18200859.9
(22) Anmeldetag: 30.04.2007
(51) Int. Cl.: H01L 51/54, C07C 13/04, C07C 13/06, C07C 13/11, C07C 13/19, C07C 49/39, C07C 49/395, C07C 49/653, C07C 255/31, C07C 255/40, C07C 255/51, C07C 261/04, C07D 213/53, C07D 333/24, C09B 11/02, C09B 47/04, C09B 57/00

(54) **DIE VERWENDUNG VON OXOKOHLENSTOFF-, PSEUDOOXOKOHLENSTOFF- UND RADIALENVERBINDUNGEN**
THE USE OF OXOCARBON, PSEUDOOXOCARBON AND RADIALENE COMPOUNDS
L'UTILISATION DE COMPOSÉ D'OXYDES DE CARBONE, DE COMPOSÉ DE PSEUDO OXYDE DE CARBONE ET DE COMPOSÉ DE RADIALÈNE

(43) Veröffentlichungstag der Anmeldung: 20.03.2019
(62) Teilanmeldung aus: 16169129.0
(73) Patentinhaber: Novaled GmbH, 01099 Dresden (DE)
(72) Erfinder: Hartmann, Horst, 01326 Dresden (DE); Lux, Andrea, 01277 Dresden (DE); Willmann, Steffen, 01277 Dresden (DE); Zeika, Olaf, 01099 Dresden (DE)
(74) Vertreter: Bittner, Thomas L.

(56) Entgegenhaltungen:
- EP-A- 1 596 445
- JP-A- 61 254 582
- KAZUKO TAKAHASHI ET AL: "NOVEL METALLIC CHARGE-TRANSFER COMPLEXES COMPOSED OF A Ú3RADIALENETYPE ACCEPTOR: A 1,2-BIS(P-BENZOQUINO)-3-Ú2-(DICYANOMETHYLE NE- 2,5-SELENOQUINOCYCLOPROPANE DERIVATIVE", ADVANCED MATERIALS, WILEY VCH, WEINHEIM, DE, Bd. 7, Nr. 7, 1. Juli 1995 (1995-07-01), Seiten 639-641, XP000520477, ISSN: 0935-9648
- IYODA M ET AL: "Novel synthesis of hexaaryl[3]radialenes via dibromo[3]dendralenes", TETRAHEDRON LETTERS, ELSEVIER, AMSTERDAM, NL, Bd. 41, Nr. 36, September 2000 (2000-09), Seiten 7059-7064, XP004208260, ISSN: 0040-4039
- TAKEHIRO CHONAN AND KAZUKO TAKAHASHI: "The Synthesis of Difluoro and Dimethyl Derivatives of 2,6- Bis(dicyanomethylene)-2,6-dihydro-4H-cyclo penta(2,1-b:3,4-b0)- dithiophen-4-one (CPDT-TCNQ) and the Conducting Properties of the Metallic Salts Based on the Dimethyl Derivative", BULLETIN OF THE CHEMICAL SOCIETY OF JAPAN, CHEMICAL SOCIETY OF JAPAN, TOKYO, JP, Bd. 77, Nr. 8, 6. August 2004 (2004-08-06) , Seiten 1487-1497, XP007902786, ISSN: 0009-2673

## Beschreibung

Die vorliegende Erfindung betrifft Oxokohlenstoff-, Pseudooxokohlenstoff- und Radialenverbindungen sowie deren Verwendung als organischer Dotand zur Dotierung eines organischen halbleitenden Matrixmaterials zur Veränderung der elektrischen Eigenschaften desselben, als Blockermaterial sowie als Ladungsinjektionsschicht und als Elektrodenmaterial. Ebenfalls betrifft die Erfindung organische halbleitende Materialien sowie elektronische Bauelemente, in denen die Oxokohlenstoff-, Pseudooxokohlenstoff- und Radialenverbindungen eingesetzt werden.

In der vorliegenden Anmeldung werden als Radialene Alicyclen bezeichnet, in denen alle Ringatome sp2-hybridisiert sind und soweit möglich exocyclische C-C-Doppelbindungen tragen, siehe auch H. Hopf und G. Maas, Angew. Chem. (1992), 8, 955. Oxokohlenstoff- und Pseudooxokohlenstoffverbindungen sind als nicht-benzoide Aromaten hinlänglich bekannt, siehe beispielsweise G. Seitz, Nachr. Chem. Tech. Lab. 28 (1980), Seiten 804-807. Die erste Oxokohlenstoff-Verbindung, das krokonsaure Kali, wurde von L. Gmelin 1825 aus Pottasche und Kohle hergestellt. Als Pseudooxokohlenstoffe werden, wie einem Fachmann auf dem Gebiet ohne weiteres bekannt ist, solche Verbindungen bezeichnet, bei denen zumindest ein Sauerstoffatom durch ein anderes Heteroatom ersetzt ist.

Kazuko Takahashi et al., Advanced Materials, 1995, 7(7), 639-641 discloses novel metallic charge-transfer complexes composed of a radialene type acceptor.

JP 61 254582 A discloses radialene compounds and use thereof as electron donor in electrically conductive materials.

Iyoda et al., Tetrahedron Letters, 2000, 41(36), 7059-7064 discloses a synthesis method for preparing radialenes via dendralenes.

EP 1 596 445 A discloses a process for doping organic semiconductors with derivatives of diiminoquinones.

Takehiro and Takahashi, Bulletin of the Chemical Society of Japan, 2004, 77(8), 1487-1497 discloses the synthesis of difluoro and dimethyl derivatives of CPDT-TCNQ and the conductive properties thereof.

Seit einigen Jahren ist bekannt geworden, dass man organische Halbleiter durch Dotierung hinsichtlich ihrer elektrischen Leitfähigkeit stark beeinflussen kann. Solche organischen halbleitenden Matrixmaterialien können entweder aus Verbindungen mit guten Elektronendonator-Eigenschaften oder aus Verbindungen mit guten Elektronenakzeptor-Eigenschaften aufgebaut werden. Zum Dotieren von Elektronendonator-Materialien (HT) sind starke Elektronen-Akzeptoren wie Tetracyanochinondimethan (TCNQ) oder 2,3,5,6-Tetrafluoro-tetracyano-1,4-benzochinondimethan (F4TCNQ) bekannt geworden, M. Pfeiffer, A. Beyer, T. Fritz, K. Leo, Appl. Phys. Lett., 73 (22), 3202-3204 (1998). und J. Blochwitz, M. Pfeiffer, T. Fritz, K. Leo, Appl. Phys. Lett., 73 (6), 729-731 (1998). Diese erzeugen durch Elektronentransferprozesse in elektronendonatorartigen Basismaterialien (Löchertransportmaterialien) sog. Löcher, durch deren Anzahl und Beweglichkeit sich die Leitfähigkeit des Basismaterials mehr oder weniger signifikant verändert. Als Matrixmaterialien mit Löchertransporteigenschaften sind beispielsweise N,N'-perarylierte Benzidine **TPD** oder N,N',N"-perarylierte Starburstverbindungen, wie die Substanz **TDATA,** oder aber auch bestimmte Metallphthalocyanine, wie insbesondere Zinkphthalocyanin **ZnPc** bekannt.

Die bisher beschriebenen Verbindungen haben jedoch für eine technische Anwendung Nachteile in der Produktion dotierter halbleitender organischer Schichten oder von entsprechenden elektronischen Bauteilen mit derartigen dotierten Schichten, da die Fertigungsprozesse in großtechnischen Produktionsanlagen oder solchen im Technikumsmaßstab nicht immer ausreichend präzise gesteuert werden können, was zu hohem Steuerungs- und Regelaufwand innerhalb der Prozesse führt, um eine gewünschte Produktqualität zu erzielen, oder zu unerwünschten Toleranzen der Produkte. Ferner bestehen Nachteile bei der Verwendung bisher bekannter organischer Donatoren bezüglich der elektronischen Bauelementstrukturen, wie Leuchtdioden (OLEDs), Feldeffekttransistor (FET) oder Solarzellen selber, da die genannten Produktionsschwierigkeiten bei der Handhabung der Dotanden zu unerwünschten Ungleichmäßigkeiten in den elektronischen Bauteilen oder unerwünschten Alterungseffekten der elektronischen Bauteile führen können. Gleichzeitig ist jedoch zu beachten, dass die zu verwendenden Dotanden extrem hohe Elektronenaffinitäten (Reduktionspotentiale) und andere für den Anwendungsfall geeignete Eigenschaften aufweisen, da beispielsweise die Dotanden unter gegebenen Bedingungen auch die Leitfähigkeit oder andere elektrische Eigenschaften der organisch halbleitenden Schicht mit bestimmen. Entscheidend für den Dotiereffekt sind die energetischen Lagen des HOMO des Matrixmaterials und des LUMO des Dotanden.

Der vorliegenden Erfindung liegt die Aufgabe zugrunde, die Nachteile des Stands der Technik zu überwinden, insbesondere neue organische mesomere Verbindungen bereitzustellen, die insbesondere als Dotand zur Dotierung organischer Halbleiter eingesetzt werden können, die ferner im Produktionsprozeß leichter handhabbar sind und die zu elektronischen Bauteilen führen, deren organische halbleitende Materialien reproduzierbar hergestellt werden können.

Diese Aufgabe wird durch die unabhängigen Ansprüche der vorliegenden Anmeldung gelöst. Bevorzugte Ausführungsformen ergeben sich aus den Unteransprüchen.

Bei den erfindungsgemäßen Verbindungen ist die Lage des LUMO so niedrig, daß weitere technisch interessante Lochtransportmaterialien jetzt erst effizient dotiert werden können. Durch die außergewöhnlich niedrige Lage des LUMO und das damit verbundene hohe Reduktionspotential der Verbindungen können auch Leistungseffizienzen von Solarzellen wesentlich verbessert werden. Zusätzlich sind diese Verbindungen aufgrund ihrer hohen Polarität außergewöhnlich diffusionsstabil in organischen Schichten. Durch höhere Verdampfungstemperatur bzw. geringere Flüchtigkeit unter gleichen Bedingungen können die Produktionsprozesse besser kontrolliert und damit mit geringerem Aufwand und reproduzierbarer durchgeführt werden, wobei durch die Bereitstellung von Oxokohlenstoffen, Pseudooxokohlenstoffen und Radialenen als Dotanden diese in den jeweiligen Bauteilen bei geringen Diffusionskoeffizienten, die zeitlich gleichbleibende Bauelementstruktur gewährleisten, eine ausreichende elektrische Leitfähigkeit der organischen halbleitenden Matrix bei günstiger Elektronenaffinität der Dotanden ermöglichen. Ferner kann durch die Dotanden die Ladungsträgerinjektion von Kontakten in die dotierte Schicht verbessert werden. Ferner kann das dotierte organische Halbleitermaterial bzw. das resultierende elektronische Bauteil aufgrund der erfindungsgemäß verwendeten Verbindungen eine verbesserte Langzeitstabilität aufweisen. Dies betrifft beispielsweise eine Verringerung der Dotandenkonzentration mit der Zeit. Ferner betrifft dies die Stabilität der dotierten Schicht, die benachbart zu undotierten Schichten eines elektrooptischen Bauteils angeordnet ist, so daß elektrooptische Bauteile mit erhöhter Langzeitstabilität der elektrooptischen Eigenschaften, wie Lichtausbeute bei einer vorgegebenen Länge, Wirksamkeit einer Solarzelle oder dergleichen, resultieren.

Die Bedampfungsrate eines Substrats mit den erfindungsgemäß verwendeten Verbindungen kann beispielsweise unter Verwendung eines Quarzdickenmonitors bestimmt wird, wie er beispielsweise bei der Herstellung von OLEDs üblicherweise eingesetzt wird. Insbesondere kann das Verhältnis der Bedampfungsraten von Matrixmaterialien und Dotanden durch unabhängige Messungen derselben unter Verwendung von zwei getrennten Quarzdickenmonitoren gemessen werden, um das Dotierungsverhältnis einzustellen.

Es versteht sich, dass die erfindungsgemäß verwendeten Verbindungen vorzugsweise derart beschaffen sind, dass sie mehr oder weniger oder praktisch unzersetzt verdampfen. Es können unter Umständen jedoch auch zielgerichtet Precursor als Dotandenquelle eingesetzt werden, die die erfindungsgemäß verwendeten Verbindungen freisetzen, beispielsweise Säureadditionssalze, beispielsweise einer flüchtigen oder nichtflüchtigen anorganischen oder organischen Säure, oder Charge-Transfer-Komplexe derselben, wobei die Säuren bzw. Elektronen-Donatoren vorzugsweise nicht oder nur gering flüchtig sind oder der Charge-Transfer-Komplex selber als Dotand wirkt.

Vorzugsweise ist der Dotand derart ausgewählt, dass er unter sonst gleichen Bedingungen wie insbesondere Dotierungskonzentration (Molverhältnis Dotand:Matrix, Schichtdicke, Stromstärke) bei gegebenem Matrixmaterial (beispielsweise Zinkphtalocyanin oder einem anderen weiter unten genannten Matrixmaterial) eine genau so hohe oder vorzugsweise eine höhere Leitfähigkeit erzeugt als F4TCNQ, beispielsweise eine Leitfähigkeit (S/cm) von größer/gleich dem 1,1-fachen, 1,2-fachen oder größer/gleich dem 1,5-fachen oder zweifachen derjenigen von F4TCNQ als Dotand.

Vorzugsweise ist der erfindungsgemäß verwendete Dotand derart ausgewählt, dass das mit diesem dotierte halbleitende organische Matrixmaterial nach einer Temperaturänderung von 100°C auf Raumtemperatur (20°C) noch ≥ 20%, vorzugsweise ≥ 30%, besonders bevorzugt ≥ 50% oder 60% der Leitfähigkeit (S/cm) des Wertes bei 100°C aufweist.

Im folgenden werden einige bevorzugte Oxokohlenstoffe, Pseudooxokohlenstoffe bzw. Radialene gezeigt, die vorteilhaft für die erfindungsgemäßen Zwecke eingesetzt werden können:

### Derivate der [3] Radialene

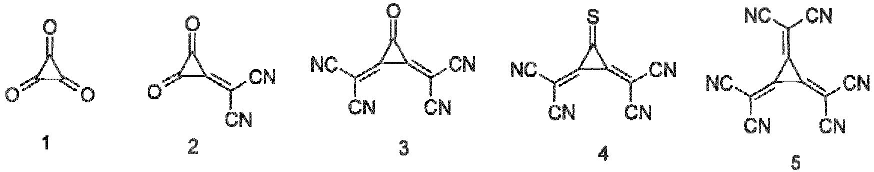

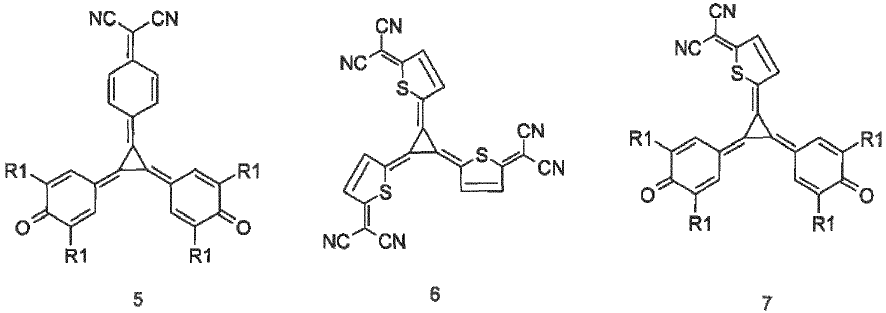

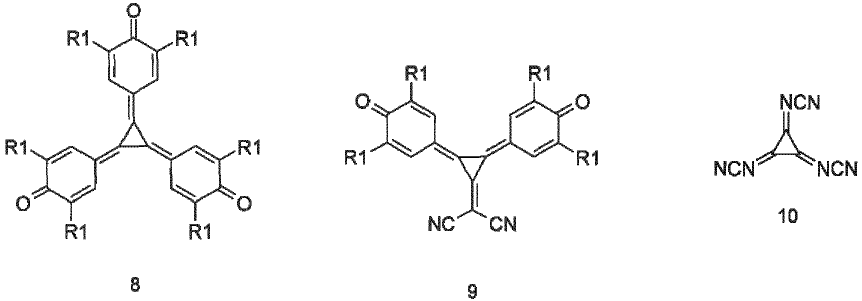

R1 = Methyl, Iso-Propyl, t-Butyl

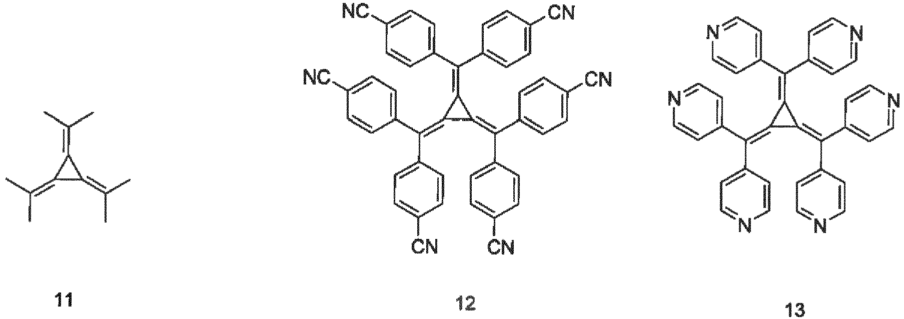

### Derivate der [4] Radialene

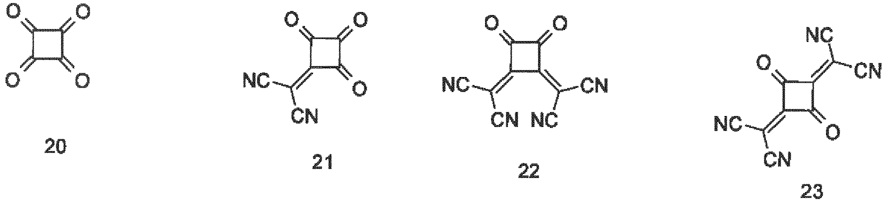

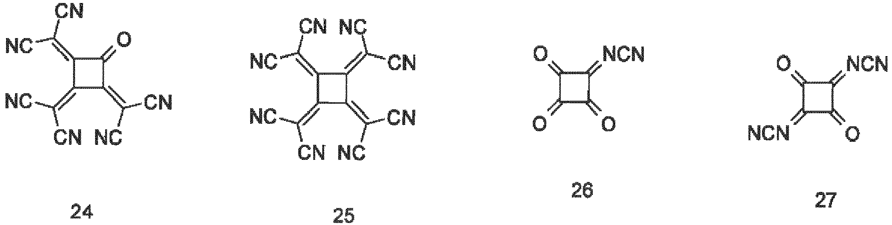

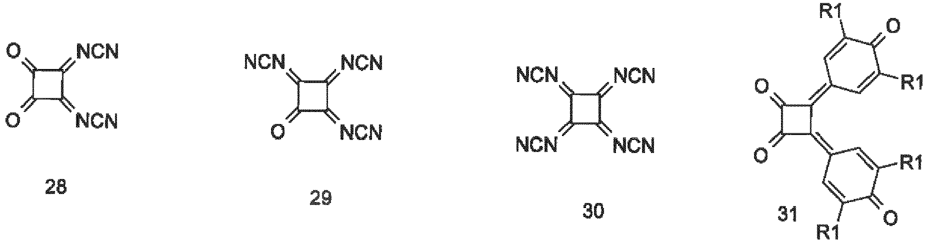

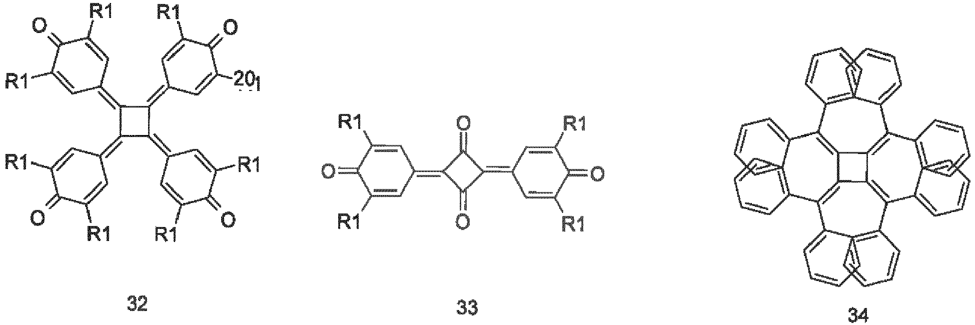

### Derivate der [5] Radialene

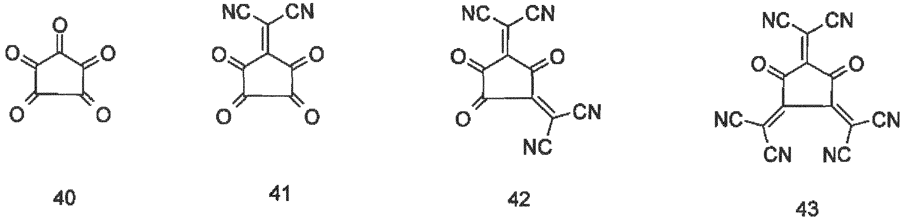

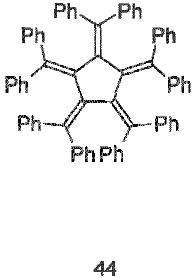

Weitere Derivate der Oxokohlenstoff-, Pseudooxokohlenstoff- bzw. [6] Radialen-Strukturen

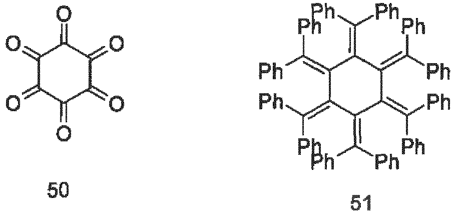

### Darstellung der Oxokohlenstoff-, Pseudooxokohlenstoff- bzw. Radialen-Strukturen

Die erste Oxokohlenstoff-Verbindung das krokonsaure Kali wurde von L.Gmelin 1825 aus Pottasche und Kohle hergestellt. Oxokohlenstoffe und deren Ester und Halogenide reagieren bevorzugt mit elektronenreichen Verbindungen, wie aliphatischen und aromatischen Aminen, Aromaten und Heteroaromaten. A.H.Schmidt, Synthesis (1980) 961. Besonders geeignet für erfindungsgemäße Anwendungen sind die Reaktionsprodukte aus Tetrachlorcyclopropen und Phenolen in Gegenwart von Lewissäuren bzw. CH-aciden Verbindungen mittels starker Basen, wie z.B. Arylacetonitrile, 1,3-Diketone, Cyclopentadiene, Malodinitrile, Akzeptorsubstituierte Diarylmethane, elektronenarme Diheteroarylmethane. Nach erfolgter Oxidation werden [3]Radialene erhalten, R.West et al. J. Org. Chem. (1975) 40 2295; T.Kazuka, T.Shinji J. Chem. Soc. Chem. Commun.(1994) 519; T.Fukunaga et al. JACS (1976) 98 610.

Weiterhin auch sehr gut geeignet sind Quadratsäuredichlorid und Phenole, die anschließend zu 4[Radialenen] oxidiert werden können, R.West, S.K.Koster J.Org.Chem. (1975) 40 2300; das nucleophile Anion des CH-aciden Malonsäuredinitrils lässt sich ebenfalls bevorzugt mit Estern unter Alkoholabspaltung zu dianionischen Quadratsäureverbindungen substituieren, T.Fukanaga J.Am.Chem.Soc. (1976) 98 610; W.Ziegenbein, H.-E.Sprenger Angew. Chem. (1967) 79 581; G.Seitz et al. Chem. Ber. (1987) 120 1691. Die Oxidation dieser CN-substituierten Verbindungen gelang bisher nur elektrochemisch, T.A.Blinka, R.West, Tetrahedron Lett. (1983) 24 1567. Durch thermische Dimerisierung von Dichinonethylenen lassen sich gleichfalls [4]Radialene darstellen, R.West,S.K.Koster, JOC (1975) 40 2300.

Von Fatiadi konnten die ersten Krokonsäurederivate hergestellt werden, die mit Malodinitril substituiert worden sind, J.Org.Chem. (1980) 45 1338, J.Am.Chem.Soc. (1978) 100 2586. Die Oxidation dieser Verbindungen wurde auch von ihm elektrochemisch untersucht, A.J.Fatiadi, L.M.Doane J.Electroanal.Chem. (1982) 135 193-209.

Es sind aber auch [6]Radialene bekannt, H.Hopf, A.K.Wick Helv. Chim. Acta (1961)46 380-6.

Einige spätere Vertreter fanden bzw. finden Anwendung in der Elektrophotographie, als Elektrolumineszenzmaterial in Bildschirmen, als Farbstoff, als Photoleiter, als organische Oxidantien, US 4003943 (1977),JP 07-168377, JP 2004010703 A (2004), US 4133821 (1979).

Darstellung neuer cyclischer Oxokohlenstoff- bzw. Pseudooxokohlenstoff-Derivate
a) 1,3 -Bis(dicyanomethylen)indan-2-yliden-bis(4-oxo-[3,5-di-t-butyl]-2,5-cyclohexadienyliden)-cyclopropan
   4,75 g Bis(4-oxo-[3,5-di-*t*-butyl]-2,5-cyclohexadienyl)-cyclopropenon, 3,5 g 1,3-Bis(dicyanomethylen)-indan sowie 60mg β-Alanin werden in 12 ml Acetanhydrid gelöst und kurz am Rückfluß unter Rühren erhitzt. Man versetzt mit 60ml Toluen, lässt abkühlen und saugt den rotbrauen kristallinen Feststoff ab. Anschließend wird mit Benzin/Toluen gewaschen und umkristallisiert.
   Ausbeute: 4,6g
   Unter Argon werden 2,5g der rotbraunen Kristalle in 100ml Chloroform gelöst und mit einer Lösung aus 4,7g rotem Blutlaugensalz und 8,8g KOH in 150 ml Wasser vereinigt. Nach 1h intensiven Rührens wird die organische Phase mit Na2SO4 getrocknet und eingeengt und das Produkt umkristallisiert.
   Ausbeute: 2,3g schwarzgrüne Kristalle Fp.: >250°C unter Zersetzung
b) Hexa(methoxycarbonyl)trimethylencyclopropan
   Zu einer Suspension aus 16,5g 50% NaH in 340ml Glyme werden unter Argon, Eiskühlung und Rühren 24g Dimethylmalonat langsam zugetropft. Danach werden 10g Tetrachlorcyclopropen in 20 ml glyme bei 0-10°C langsam zugetropft. Die resultierende gelbe Lösung wird noch 1,5 h bei RT gerührt. Der gelbe Niederschlag wird abgesaugt und gewaschen. Das Produkt wird in Wasser gelöst und mit HCl ausgefällt, filtriert, mit Wasser gewaschen und getrocknet sowie aus Tetrachlorkohlenstoff umkristallisiert.
   Ausbeute: 60%, Fp.: 121-3°C
   2g des Produktes werden portionsweise zu einer gerührten Lösung aus 2g Natriumperiodat in 100ml Wasser gegeben und weiterhin gerührt. Den leuchtend gelben Niederschlag mit Methylenchlorid extrahieren, über Magnesiumsulfat trocknen und das Lösungsmittel abziehen. Den Rückstand in Ether aufnehmen, filtrieren und mit Ether waschen sowie aus n-Butylchlorid umtristallisieren.
   Ausbeute: 67%, Fp.: 138-40°C

### Matrixmaterialien

In der vorliegenden Erfindung werden geeignete Dotanden für organische halbleitende Materialien wie Lochtransportmaterialen HT beschrieben, die üblicherweise in OLEDs oder organischen Solarzellen verwendet werden. Die halbleitenden Materialien sind vorzugsweise intrinsisch lochleitend. Für erfindungsgemäße Dotanden des Oxokohlenstoff- bzw. des Pseudooxokohlenstoff-Typs kann das Folgende gelten.

Das Matrixmaterial kann teilweise (> 10 oder > 25 Gew.-%) oder im Wesentlichen (> 50 Gew.-% oder > 75 Gew.-%)) oder vollständig bestehen aus einem Metallphtalocyanin-Komplex, einem Porphyrin-Komplex, insbesondere Metallporphyrinkomplex, einer Oligothiophen-, Oligophenyl-, Oligophenylenvinylen- oder Oligofluoren-Verbindung, wobei das Oligomere vorzugsweise 2-500 oder mehr, vorzugsweise 2-100 oder 2-50 oder 2-10 monomere Einheiten umfasst. Gegebenenfalls kann das Oligomer auch > 4, > 6 oder > 10 oder mehr monomere Einheiten umfassen, insbesondere auch für die oben angegebenen Bereiche, also beispielsweise 4 oder 6-10 monomere Einheiten, 6 oder 10-100 monomere Einheiten oder 10-500 monomere Einheiten. Die Monomere bzw. Oligomere können substituiert oder unsubstituiert sein, wobei auch Block- oder Mischpolymerisate aus den genannten Oligomeren vorliegen können, sowie eine Verbindung mit einer Triarylamin-Einheit oder eine Spiro-Bifluoren-Verbindung. Die genannten Matrixmaterialien können auch in Kombination miteinander vorliegen, gegebenenfalls auch in Kombination mit anderen Matrixmaterialien. Die Matrixmaterialien können elektronenschiebende Substituenten wie Alkyl- oder Alkoxy-Reste aufweisen, die eine verminderte Ionisierungsenergie aufweisen oder die Ionisierungsenergie des Matrixmaterials vermindern.

Die als Matrixmaterial eingesetzten Metallphtalocyaninkomplexe oder Porphyrinkomplexe können ein Hauptgruppenmetallatom oder Nebengruppenmetallatom aufweisen. Das Metallatom Me kann jeweils 4-, 5- oder 6-fach koordiniert sein, beispielsweise in Form von Oxo- (Me=O), Dioxo- (O=Me=O), Imin-, Diimin-, Hydroxo-, Dihydroxo-, Amino- oder Diaminokomplexen, ohne hierauf beschränkt zu sein. Der Phtalocyaninkomplex oder Porphyrinkomplex kann jeweils teilweise hydriert sein, wobei jedoch vorzugsweise das mesomere Ringsystem nicht gestört wird. Die Phthalocyaninkomplexe können als Zentralatom beispielsweise Magnesium, Zink, Eisen, Nickel, Kobalt, Magnesium, Kupfer oder Vanadyl (= VO) enthalten. Die gleichen oder andere Metallatome bzw. Oxometallatome können im Falle von Porphyrinkomplexen vorliegen.

Insbesondere können solche dotierbaren Lochtransportmaterialen HT arylierte Benzidine, beispielsweise N,N'-perarylierte Benzidine oder andere Diamine wie des Typs TPD (wobei eine, mehrere oder sämtliche der Arylgruppen aromatische Heteroatome aufweisen können), geeignete arylierte Starburst-Verbindungen wie N,N',N"-perarylierte Starburstverbindungen, wie die Verbindung **TDATA** (wobei eine, mehrere oder sämtliche der Arylgruppen aromatische Heteroatome aufweisen können), sein. Die Arylreste können insbesondere für jede der oben genannten Verbindungen Phenyl, Naphthyl, Pyridin, Chinolin, Isochinolin, Peridazin, Pyrimidin, Pyrazin, Pyrazol, Imidazol, Oxazol, Furan, Pyrrol, Indol oder dergleichen umfassen. Die Phenylgruppen der jeweiligen Verbindungen können durch Thiophengruppen teilweise oder vollständig ersetzt sein.

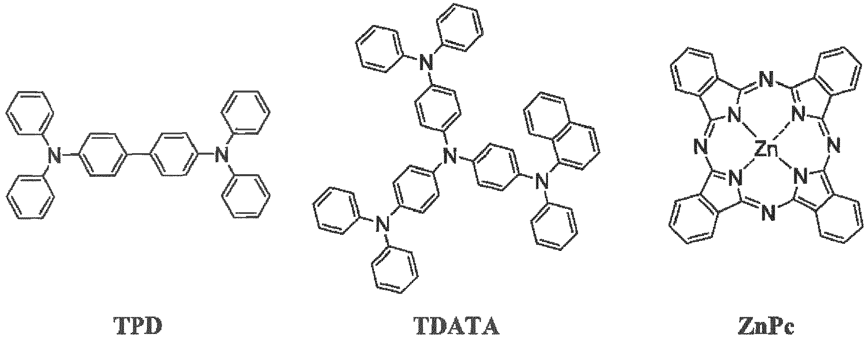

Vorzugsweise besteht das verwendete Matrixmaterial vollständig aus einem Metallphtalocyanin-Komplex, einem Porphyrin-Komplex, einer Verbindung mit einer Triarylamin-Einheit oder einer Spiro-Bifluoren-Verbindung.

Es versteht sich, dass auch geeignete andere organische Matrixmaterialien, insbesondere lochleitende Materialien verwendet werden können, die halbleitende Eigenschaften aufweisen.

### Dotierung

Die Dotierung kann insbesondere derart erfolgen, dass das molare Verhältnis von Matrixmolekül zu Dotand oder im Falle von oligomeren Matrixmaterialien das Verhältnis von Matrixmonome-renanzahl zu Dotand 1:100000, vorzugsweise 1:1 bis 1:10000, besonders bevorzugt 1:5 bis 1:1000 beispielsweise 1:10 bis 1:100, beispielsweise ca. 1:50 bis 1:100 oder auch 1:25 bis 1:50 beträgt.

### Verdampfung der Dotanden

Die Dotierung des jeweiligen Matrixmaterials (hier vorzugsweise angegeben als löcherleitendes Matrixmaterial HT) mit den erfindungsgemäß zu verwendenden Dotanden kann durch eines oder eine Kombination der folgenden Verfahren hergestellt wird:
a) Mischverdampfung im Vakuum mit einer Quelle für HT und einer für den Dotanden.
b) Sequentielles Deponieren von HT und Dotand mit anschliessender Eindiffusion des Dotanden durch thermische Behandlung
c) Dotierung einer HT-Schicht durch eine Lösung von Dotanden mit anschliessendem Verdampfen des Lösungsmittels durch thermische Behandlung
d) Oberflächendotierung einer HT-Schicht durch eine oberflächlich aufgebrachte Schicht von Dotanden

Die Dotierung kann derart erfolgen, dass der Dotand aus einer Precursor-Verbindung heraus verdampft wird, die beim Erhitzen und/oder Bestrahlung den Dotanden freisetzt. Die Bestrahlung kann mittels elektromagnetischer Strahlung, insbesondere sichtbarem Licht, UV-Licht oder IR-Licht erfolgen, beispielsweise jeweils Laserlicht, oder auch durch andere Strahlungsarten. Durch die Bestrahlung kann im wesentlichen die zur Verdampfung notwendige Wärme bereitgestellt werden, es kann auch gezielt in bestimmte Banden der zu verdampfenden Verbindungen bzw. Precursor oder Verbindungskomplexe wie Charge-Transfer-Komplexe eingestrahlt werden, um beispielsweise durch Überführung in angeregte Zustände die Verdampfung der Verbindungen durch Dissoziation der Komplexe zu erleichtern. Es versteht sich, dass die nachfolgend beschriebenen Verdampfungsbedingungen sich auf solche ohne Bestrahlung richten und für Vergleichszwecke einheitliche Verdampfungsbedingungen heranzuziehen sind.

Als Precursorverbindungen können beispielsweise zum Einsatz kommen:
a) Gemische oder stöchiometrische oder mischkristalline Verbindungen aus dem Dotand und einer inerten, nicht-flüchtigen Substanz, z.B. einem Polymer, Molsieb, Aluminiumoxid, Kieselgel, Oligomeren oder einer anderen organischen oder anorganischen Substanz mit hoher Verdampfungstemperatur, wobei der Dotand vorwiegend durch van-der-Waals Kräfte und/oder Wasserstoffbrückenbindung an dieser Substanz gebunden ist.
b) Gemisch oder stöchiometrische oder mischkristalline Verbindung aus dem Dotanden und einer mehr oder weniger Elektronendonor-artigen, nicht flüchtigen Verbindung V, wobei ein mehr oder weniger vollständiger Ladungstransfer zwischen dem Dotanden und der Verbindung V auftritt, wie in Charge-Transfer-Komplexen mit mehr oder weniger elektronenreichen Polyaromaten oder Heteroaromaten oder einer anderen organischen oder anorganischen Substanz mit hoher Verdampfungstemperatur.
c) Gemisch oder stöchiometrische oder mischkristalline Verbindung aus dem Dotanden und einer Substanz, welche zusammen mit dem Dotanden verdampft und eine gleiche oder höhere Ionisierungsenergie aufweist wie die zu dotierende Substanz HT, so dass die Substanz in dem organischen Matrixmaterial keine Haftstelle für Löcher bildet. Hierbei kann die Substanz erfindungsgemäß auch mit dem Matrixmaterial identisch sein, beispielsweise ein Metallphtalocyanin oder Benzidin-Derivat darstellen. Weitere geeignete flüchtige Co-Substanzen, wie Hydrochinone, 1,4-Phenylendiamine oder 1-Amino-4-hydroxybenze oder sonstige Verbindungen bilden Chinhydrone oder andere Charge-Transfer-Komplexe.

### Elektronisches Bauelement

Unter Verwendung der erfindungsgemäßen organischen Verbindungen zur Herstellung dotierter organischer halbleitender Materialien, die insbesondere in Form von Schichten oder elektrischen Leitungspfaden angeordnet sein können, können eine Vielzahl elektronischer Bauelemente oder diese enthaltende Einrichtungen hergestellt werden. Insbesondere können die erfindungsgemäßen Dotanden zur Herstellung von organischen lichtemitierenden Dioden (OLED), organischen Solarzellen, organischen Dioden, insbesondere solchen mit hohem Gleichrichtungsverhältnis wie 10³-10⁷, vorzugsweise 10⁴-10⁷ oder 10⁵-10⁷ oder organischen Feldeffekttransistoren verwendet werden. Durch die erfindungsgemäßen Dotanden kann die Leitfähigkeit der dotierten Schichten und/oder die Verbesserung der Ladungsträgerinjektion von Kontakten in die dotierte Schicht verbessert werden. Insbesondere bei OLEDs kann das Bauelement eine pin-Struktur oder eine inverse Struktur aufweisen, ohne hierauf beschränkt zu sein. Die Verwendung der erfindungsgemäßen Dotanden ist jedoch auf die oben genannten vorteilhaften Ausführungsbeispiele nicht beschränkt.

### Ausführungsbeispiele

Die Erfindung soll an einigen Ausführungsbeispielen näher erläutert werden.

Die erfindungsgemaß zu verwendenden Verbindungen, insbesondere die vorstehend beispielhaft angegebenen Verbindungen aus der vorstehend beschriebenen Stoffklasse der Oxokohlen- bzw. der Pseudooxokohlenstoffverbindungen werden nun in folgender Weise als Dotanden für verschiedene Lochleiter verwendet, die ihrerseits zum Aufbau bestimmter mikroelektronischer oder optoelektronischer Bauelemente, wie z.B. einer OLED, benutzt werden. Hierbei können die Dotanden gleichzeitig nebeneinander mit dem Lochtransportmaterialien der Matrix im Hochvakuum (ca. 2x 10⁻⁴Pa) bei erhöhten Temperaturen verdampft werden. Eine typische Substratbedampfungsrate für das Matrixmaterial ist 0,2 nm/s (Dichte ca. 1,5 g/cm³). Die Bedampfungsraten für die Dotanden können variieren zwischen 0.001 und 0.5 nm/s bei gleicher angenommener Dichte, jeweils entsprechend dem gewünschten Dotierungsverhältnis.

In den folgenden Beispielen wurden die Strommessungen über einen 1 mm langen und ca. 0,5 mm breiten Strompfad aus dem dotierten HT-Material bei 1V durchgeführt. Unter diesen Bedingungen leitet ZnPc praktisch keinen elektrischen Strom.

### Beispiele:

### Beispiel 1

Dotierung von ZnPc mit Dicyanomethylenbis(4-oxo-[3,5-di-*t*-butyl]-2,5-cyclohexadienyliden)cyclopropan
Leitfähigkeit: 1,5 x 10⁻⁵S/cm

### Beispiel 2

Dotierung von Spiro-TTP mit Dicyanomethylenbis(4-oxo-[3,5-di-*t*-butyl]-2,5-cyclohexadienyliden)cyclopropan
Leitfähigkeit: 3,6 x 10⁻⁷S/cm

### Beispiel 3

Dotierung von ZnPC mit 1,3-Bis(dicyanomethylen)indan-2-yliden-bis(4-oxo-[3,5-di-*t*-butyl]-2,5-cyclohexadienyliden)cyclopropan Leitfähigkeit: 5,8 x 10⁻⁶S/cm

### Beispiel 4

Dotierung von Spiro-TTP mit 1,3-Bis(dicyanomethylen)indan-2-yliden-bis(4-oxo-[3,5-di-*t-*butyl]-2,5-cyclohexadienyliden)cyclopropan Leitfähigkeit: 5 x 10⁻⁷S/cm

Die in der vorstehenden Beschreibung und in den Ansprüchen offenbarten Merkmale der Erfindung können sowohl einzeln als auch in jeder beliebigen Kombination für die Verwirklichung der Erfindung in ihren verschiedenen Ausführungsformen wesentlich sein.

## Patentansprüche

1. Verwendung einer organischen mesomeren Verbindung als Ladungsinjektionsschicht, **dadurch gekennzeichnet, daß** die mesomere Verbindung eine Oxokohlenstoff-, Pseudooxokohlenstoff- oder Radialenverbindung der folgenden Formel ist: 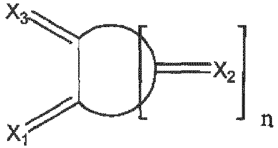 wobei n = 1-4 ist; jedes X₁, X₂, und X₃, jeweils unabhängig ausgewählt wird aus der Gruppe bestehend aus C(CN)₂, (CF₃)C(CN), (NO₂)C(CN), C(Halogen)₂, C(CF₃)₂, NCN, O, S, NR₁, 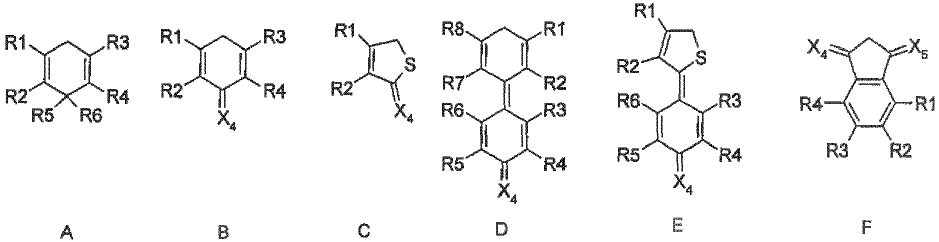 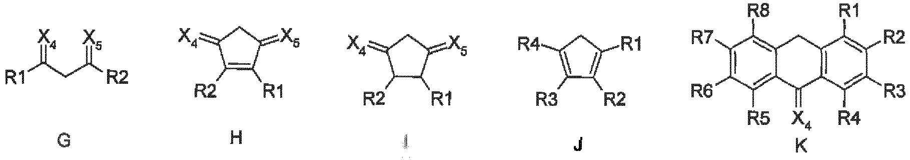 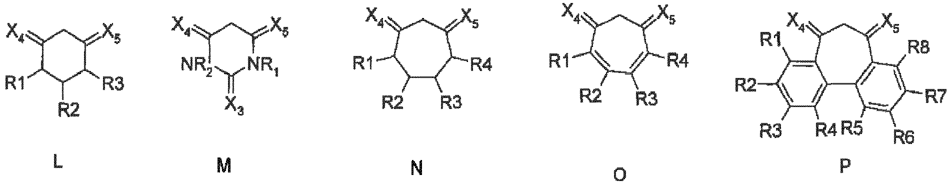 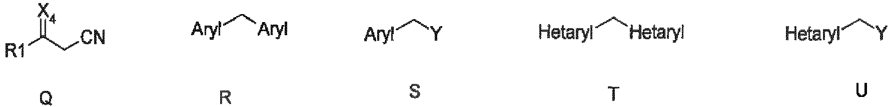 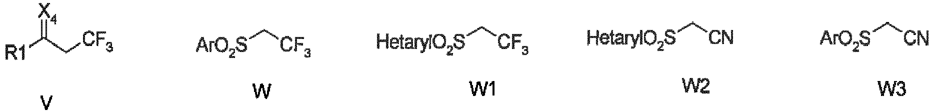 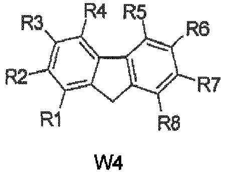 wobei X₄ und X₅ jeweils unabhängig ausgewählt werden aus der Gruppe, bestehend aus C(CN)₂, (CF₃)C(CN), (NO₂)C(CN), C(Halogen)₂, C(CF₃)₂, NCN, O, S oder NR₁,
wobei Y = CN, NO₂, COR₁ oder perhalogeniertes Alkyl ist; Aryl bzw. Ar ein substituierter oder unsubstituierter aromatischer Kohlenwasserstoff oder Biaryl ist, ggf. polycyclisch; Hetaryl eine substituierte oder unsubstituierte aromatische heterocyclische Verbindung oder Biheteroaryl ist, vorzugsweise elektronenarm, ggf. mehrkernig oder teilweise oder vollständig hydriert bzw. fluoriert; und R₁-R₈ unabhängig ausgewählt werden aus Wasserstoff, Halogen, CN, NO₂, COR₁, Alkyl, Alkoxy, Aryl und Heteroaryl.

2. Verwendung nach Anspruch 1, **dadurch gekennzeichnet, daß** Y Perfluoralkyl, wie CF₃, ist.

3. Verwendung nach Anspruch 1 oder 2, **dadurch gekennzeichnet, daß** Aryl teilweise oder vollständig fluoriert ist.

4. Verwendung nach einem der vorangehenden Ansprüche, **dadurch gekennzeichnet, daß** Hetaryl ausgewählt wird aus Pyridyl, Pyrimidyl, Triazin und Oxadiazol.

5. Verwendung nach einem der vorangehenden Ansprüche, **dadurch gekennzeichnet, daß** R₁-R₈ unabhängig ausgewählt werden aus perhalogenierten und/oder teilweise halogenierten Alkylgruppen, insbesondere perfluorierten Alkylgruppen.

6. Elektronisches Bauelement mit einem elektronisch funktionell wirksamen Bereich, **dadurch gekennzeichnet, daß** der elektronisch wirksame Bereich eine Ladungsinjektionsschicht umfasst, die aus zumindest einer Oxokohlenstoff-, Pseudooxokohlenstoff- und Radialenverbindung, wie in einem der Ansprüche 1 bis 5 definiert, besteht.

7. Elektronisches Bauelement nach Anspruch 6 in Form einer organischen lichtemittierenden Diode, einer photovoltaischen Zelle, einer organischen Solarzelle, einer organischen Diode oder eines organischen Feldeffekttransistors.

## Claims

1. Use of an organic mesomeric compound as a charge injection layer, **characterized in that** the mesomeric compound is an oxocarbon, a pseudo-oxocarbon or a radialene compound with the following formula: 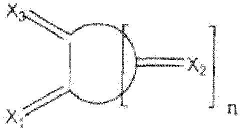 in which n = 1-4; each X₁, X₂ and X₃ is respectively independently selected from the group consisting of C(CN)₂, (CF₃)C(CN), (NO₂)C(CN), C(halogen)₂, C(CF₃)₂, NCN, O, S, NR₁, 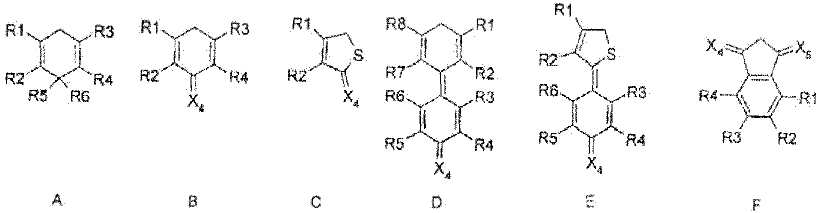 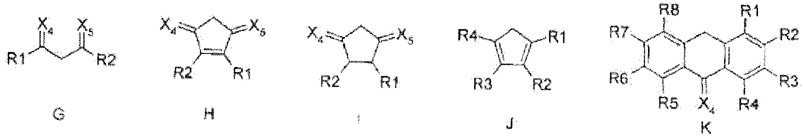 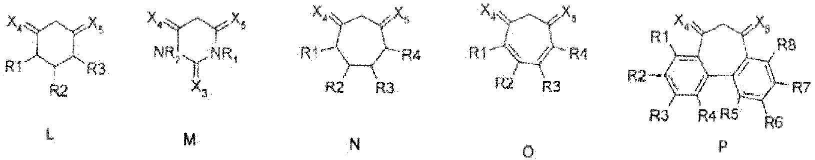 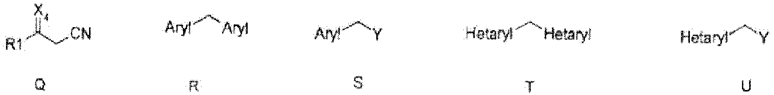 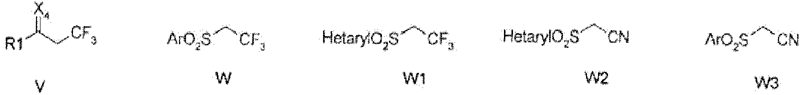 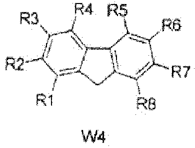 wherein X₄ and X₅ are respectively independently selected from the group consisting of C(CN)₂, (CF₃)C(CN), (NO₂)C(CN), C(halogen)₂, C(CF₃)₂, NCN, O, S or NR₁,
wherein Y = CN, NO₂, COR₁ or is perhalogenated alkyl; aryl, respectively Ar is a substituted or unsubstituted aromatic hydrocarbon or biaryl, or optionally polycyclic; hetaryl is a substituted or unsubstituted aromatic heterocyclic compound or biheteroaryl, preferably electron-deficient, optionally polynucleic or partially or completely hydrated or fluorinated; and R₁-R₈ are independently selected from hydrogen, halogen, CN, NO₂, COR₁, alkyl, alkoxy, aryl and heteroaryl.

2. Use as claimed in claim 1, **characterized in that** Y is perfluoroalkyl, such as CF₃.

3. Use as claimed in claim 1 or claim 2, **characterized in that** the aryl is partially or completely fluorinated.

4. Use as claimed in one of the preceding claims, **characterized in that** the hetaryl is selected from pyridyl, pyrimidyl, triazine and oxadiazole.

5. Use as claimed in one of the preceding claims, **characterized in that** R₁-R₈ are independently selected from perhalogenated and/or partially halogenated alkyl groups, in particular perfluorinated alkyl groups.

6. An electronic component with an electronically functionally active region, **characterized in that** the electronically active region comprises a charge injection layer which consists of at least one oxocarbon, pseudo-oxocarbon and radialene compound as defined in one of claims 1 to 5.

7. The electronic component as claimed in claim 6, in the form of an organic light emitting diode, a photovoltaic cell, an organic solar cell, an organic diode or an organic field effect transistor.

## Revendications

1. Utilisation d'un composé organique mésomère comme couche d'injection de charge, **caractérisée en ce que** le composé mésomère est un composé d'oxocarbone, un composé de pseudo-oxocarbone ou un composé radialène de la formule suivante : 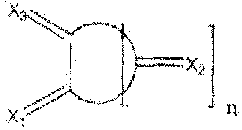 dans laquelle n = 1-4 ; chaque X₁, X₂ et X₃ est choisi chaque fois indépendamment dans le groupe constitué de C(CN)₂, (CF₃)C(CN), (NO₂)C(CN), C(halogène)₂, C(CF₃)₂, NCN, O, S, NR₁, 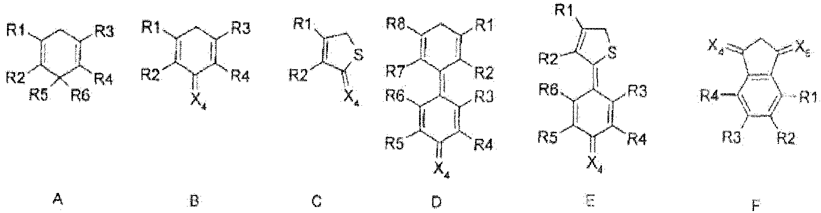 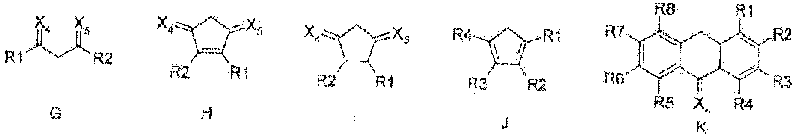 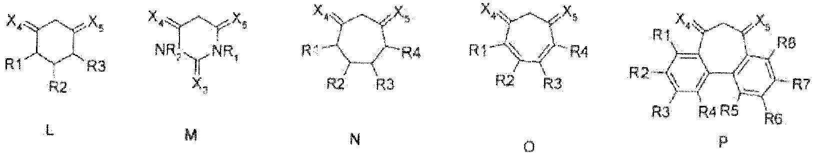 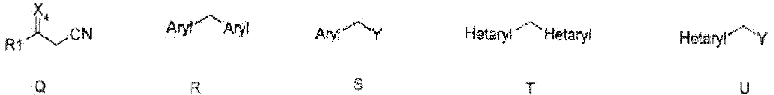 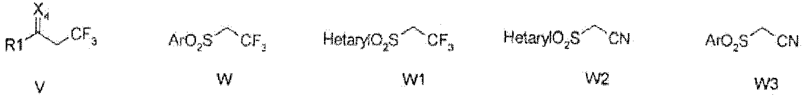 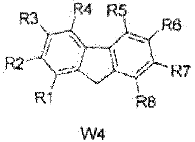 X₄ et X₅ étant choisis chaque fois indépendamment dans le groupe constitué de C(CN)₂, (CF₃)C(CN), (NO₂)C(CN), C(halogène)₂, C(CF₃)₂, NCN, O, S ou NR₁,
dans laquelle Y = CN, NO₂, COR₁ ou de l'alkyle perhalogéné ; l'aryle respectivement Ar étant un hydrocarbure aromatique substitué ou non substitué ou un biaryle, polycyclique le cas échéant ; l'hétéroaryle étant un composant aromatique hétérocyclique substitué ou non substitué ou un bihétéroaryle, de préférence déficient en électrons, le cas échéant polynucléique ou partiellement ou totalement hydrogéné ou fluoré ; et R₁ à R₈ étant choisis indépendamment parmi un hydrogène, un halogène, un CN, un NO₂, un COR₁, un alkyle, un alkoxy, un aryle et un hétéroaryle.

2. Utilisation selon la revendication 1, **caractérisée en ce que** Y est un perfluoroalkyle, comme CF₃.

3. Utilisation selon la revendication 1 ou 2, **caractérisée en ce que** l'aryle est partiellement ou totalement fluoré.

4. Utilisation selon l'une quelconque des revendications précédentes, **caractérisée en ce que** l'hétéroaryle est choisi parmi un pyridyle, un pyrimidyle, une triazine et un oxadiazole.

5. Utilisation selon l'une quelconque des revendications précédentes, **caractérisée en ce que** les R₁ à R₈ sont choisis indépendamment dans des groupes alkyle perhalogénés et/ou partiellement halogénés, notamment dans des groupes alkyle perfluorés.

6. Composant électronique, pourvu d'une zone à efficacité fonctionnelle électronique, **caractérisé en ce que** la zone électroniquement efficace comprend une couche d'injection de charge qui est constituée d'au moins un composé d'oxocarbone, un composé de pseudo-oxocarbone et un composé radialène, tels que définis dans l'une quelconque des revendications 1 à 5.

7. Composant électronique selon la revendication 6, sous la forme d'une diode organique émettrice de lumière, d'une cellule photovoltaïque, d'une cellule solaire organique, d'une diode organique ou d'un transistor organique à effet de champ.
